# EUROPEAN PATENT APPLICATION

(11) **EP 4 663 100 A1**
(43) Date of publication of application: **17.12.2025**
(21) Application number: 25178218.1
(22) Date of filing: 22.05.2025
(51) Int. Cl.: A61B 5/01, A61B 5/00

(54) **METHOD AND OPTICAL APPARATUS FOR TRACKING CORE BODY TEMPERATURE**

(30) Priority: 13.06.2024 FI 20245752
(71) Applicant: Pixieray Oy, 02630 Espoo (FI)
(72) Inventor: Virtanen, Juha, 00560 Helsinki (FI); Pitkänen, Ari, 01200 Vantaa (FI)
(74) Representative: Moosedog Oy

(57) **Abstract**

Disclosed is a method for tracking a core body temperature of a user using an optical apparatus (200). The method comprising measuring a temperature of at least one point (P1, P2, P3, P4, P5, P6) on an ocular surface of the user's eye, at a plurality of time instants during a time period, wherein said measuring is performed using at least one temperature sensor (204A, 204B, 302), and wherein the at least one point lies within a sensing area (304) of the at least one temperature sensor; and estimating the core body temperature of the user at the plurality of time instants, based on the measured temperature of the at least one point at the plurality of time instants.

## Description

### TECHNICAL FIELD

The present disclosure relates to methods for tracking core body temperatures of users using optical apparatuses. Moreover, the present disclosure relates to optical apparatuses.

### BACKGROUND

Core Body Temperature (CBT) is a critical physiological parameter often associated with various health conditions, including fever, circadian rhythm disruptions, and hormonal changes such as those observed during the ovulation cycle and menopause. Monitoring the CBT continuously and unobtrusively significantly benefits users by enabling early detection of illnesses and facilitating the tracking of their biological rhythms. While the CBT is an essential indicator of overall health, its precise measurement presents challenges due to the inherent variability of temperatures among different organs within the human body. Although, various methods have been employed to estimate the CBT indirectly, including measuring temperatures at surrogate sites such as the rectum, axilla, tympanic membrane, or sublingual region, but such methods are often impractical for continuous monitoring, limiting their utility in capturing dynamic changes in the CBT over time.

The present solutions in wearable technology have introduced adhesive patches designed to measure axillary temperature, providing a potential solution for continuous monitoring of the CBT. However, prolonged use of adhesive patches is not desirable for individuals over extended periods. Other present solutions include patches that estimate the CBT based on skin temperature and heat flux, as well as ingestible smart pills capable of reporting intestinal temperatures. While such present solutions offer promising insights into temperature monitoring, they are not suitable for long-term continuous use due to practical limitations or discomfort associated with their usage. Moreover, existing wearable devices, such as the Oura ring, measure skin temperature under controlled conditions, but face challenges related to unreliable skin contact and environmental variations. However, there still exists a need for devices capable of measuring the CBT or changes in the CBT during daytime activities. Furthermore, some solutions attempt to integrate skin temperature sensors into eyeglasses but such solutions also face the challenges related to the unreliable skin contact and the environmental variations, limiting their effectiveness in accurately estimating the CBT or detecting changes in the CBT over longer durations of time.

Therefore, in light of the foregoing discussion, there exists a need to overcome the aforementioned drawbacks.

### SUMMARY

The aim of the present disclosure is to provide a method and an optical apparatus for tracking a core body temperature of a user to estimate and analyse trends in the core body temperature of the user over a long period of time. The aim of the present disclosure is achieved by a method and an optical apparatus for tracking a core body temperature of a user as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 is an illustration of a flowchart for depicting steps of a method for tracking a core body temperature of a user using an optical apparatus, in accordance with an embodiment of the present disclosure;
FIG. 2 is a schematic illustration of an optical apparatus, in accordance with an embodiment of the present disclosure; and
FIG. 3 is a schematic illustration of a user's eye when an optical apparatus is in use, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for tracking a core body temperature of a user using an optical apparatus, the method comprising:
measuring a temperature of at least one point on an ocular surface of the user's eye, at a plurality of time instants during a time period, wherein said measuring is performed using at least one temperature sensor, and wherein the at least one point lies within a sensing area of the at least one temperature sensor; and
estimating the core body temperature of the user at the plurality of time instants, based on the measured temperature of the at least one point at the plurality of time instants.

The present disclosure provides an aforementioned method that is able to track the core body temperature of the user effectively and continuously for the time period. Moreover, the method provides a simple and convenient way for estimating the core body temperature of the user in a non-contact and easy to implement manner. Furthermore, the tracking of the core body temperature by the method enables to continuously monitor physiological conditions and gain useful insights into health parameters of the user's body.

In a second aspect, the present disclosure provides an optical apparatus comprising:
a frame;
at least one temperature sensor arranged on the frame, wherein when the optical apparatus is in use, the at least one temperature sensor faces a user's eye; and
at least one processor coupled to the at least one temperature sensor, wherein the at least one processor is configured to:
   control the at least one temperature sensor to measure a temperature of at least one point on an ocular surface of a user's eye, at a plurality of time instants during a time period, wherein the at least one point lies within a sensing area of the at least one temperature sensor; and
   estimate the core body temperature of the user at the plurality of time instants, based on the measured temperature of the at least one point at the plurality of time instants.

The present disclosure provides an aforementioned optical apparatus that is able to track the core body temperature of the user effectively and continuously for the time period. Moreover, the at least one temperature sensor in the optical apparatus provides a simple and convenient way for estimating the core body temperature of the user in a non-contact and easy to implement manner. Furthermore, the tracking of the core body temperature by the optical apparatus enables to continuously monitor physiological conditions during activities in various conditions and gain useful insights into health parameters of the user's body.

Throughout the present disclosure, the term *"core body temperature"* refers to an internal temperature of the body of the user. The core body temperature being in a range of 36.5°C and 37.5°C is considered to be of a normal level. Notably, keeping the core body temperature stable is crucial for the body of the user to function properly, whereas significant changes in the core body temperature have adverse effects on the body of the user. Subsequently, tracking the core body temperature of the user over an entire duration of the time period allows to gain valuable insights into health conditions of the user.

Throughout the present disclosure, the term *"ocular surface of the user's eye"* refers to a surface around the user's eye that comprises at least one of: a corneal surface of the user's eye, a surrounding skin surface of the corneal surface, an eyelid surface. Herein, the term *"surrounding skin surface of the corneal surface"* refers to the skin surface that surrounds a periphery of the corneal surface. The temperature of the ocular surface of the user's eye has a linear correlation with the core body temperature, and thus, the core body temperature of the user can be estimated using the temperature of the ocular surface of the user's eye. Throughout the present disclosure, the term *"at least one point"* refers to one or more points that lie on the ocular surface of the user's eye. Subsequently, measuring the temperature of the at least one point on the ocular surface of the user's eye enables to determine the temperature of the ocular surface of the user's eye.

Throughout the present disclosure, the term *"time period"* refers to a predefined duration of time for which the core body temperature of user is to be tracked. For example, the time period is of 1 hour, 5 hrs, 10 hrs, 1 day and the like. Throughout the present disclosure, the term *"plurality of time instants"* refers to multiple different instances of time spread across the entire duration of the time period. Subsequently, measuring the temperature of the at least one point on the ocular surface of the user's eye at the plurality of time instants enables to track the temperature of the ocular surface of the user's eye over the entire duration of the time period. Optionally, time interval between the plurality of time instants varies based on a current phase of the day (i.e., morning, evening, night, and the like). For example, in morning the time interval between the plurality of time instants is of 10 minutes, while in evening the time interval between the plurality of time instants is of 30 minutes, for the time period of 1 day.

Optionally, the method further comprising:
processing eye-tracking data for determining a blink pattern of the user's eye; and
determining a time instant of measuring the temperature of the at least one point, based on the blink pattern, wherein said time instant lies within an inter-blink interval.

In this regard, the eye-tracking data comprises data that is related to and is indicative of a blinking state of the user's eye and/or a non-blinking state of the user's eye. The blinking state of the eye encompasses a beginning of the blink of the user's eye, a duration of the blink of the user's eye, and an ending of the blink of the user's eye. The eye-tracking data corresponding to each of these stages of the blinking state of the user's eye is different and distinctive from each other. Notably, the eye-tracking data is collected using eye-tracking unit. Herein, the eye-tracking data may include the images of the user's eye, and the beginning of the blink of the user's eye may be detected when certain features (for example, such as portions of cornea lying above pupils) of the user's eye, start to become invisible (for example, be only partially visible) in the images of the user's eye.

Throughout the present disclosure, the term *"blink pattern of the user's eye*" refers to a pattern that indicates a time-based mathematical relationship between those instances of time at which the user's eye has blinked. In other words, the blink pattern is indicative of what are the time instances when the user's eye blinks. Optionally, the blink pattern is indicative of at least one of: a blink rate, a blink duration. Notably, the blink pattern of the user's eye enables to determine future instances of time at which the user's eye will blink. It will be appreciated that the eye-tracking data is indicative of the blink pattern of the user's eye and thus, processing the eye-tracking data enables to determine the blink pattern of the user's eye. Optionally, the eye-tracking data is processed by employing at least one of: an image processing algorithm, a feature extraction algorithm, a data processing algorithm. Other suitable algorithm(s) can also be employed, depending on a type of the eye-tracking data.

Throughout the present disclosure, the term *"inter-blink interval"* refers to an interval of time present between two consecutive blinks of the user's eye. It may be appreciated that during the inter-blink interval the eye of the user is open, i.e., the user is in a state where the eyelid is fully open, allowing direct visibility of the ocular surface. For example, the inter-blink interval for the user's eye is 5 seconds. It will be appreciated that the inter-blink interval is determined by processing and analyzing the blink pattern of the user's eye. Throughout the present disclosure, the term *"time instant of measuring the temperature"* refers to a specific moment of time at which the temperature of the at least one point is measured. It will be appreciated that the time instant is amongst the plurality of time instants during the time period. Notably, the time instant lying within the inter-blink interval ensures that the temperature of the at least one point is measured when the user's eye is not blinking. Subsequently, the time instant of measuring the temperature of the at least one point is determined based on the blink pattern to ensure that the time instant lies within the inter-blink interval. A technical effect is that the temperature of at least one point is measured only when the user's eye is not blinking, which significantly increases the accuracy of estimating the core body temperature, since the temperature measured during the blink of the user's eye is error-prone and thus, lead to incorrect estimation of the core body temperature.

Throughout the present disclosure, the term *"temperature sensor"* refers to a sensing device that is able to sense a temperature of any surface. Notably, the at least one temperature sensor is used for measuring the temperature of at least one point on the ocular surface of the user's eye at the plurality of time instants during the time period. For example, the at least one temperature sensor is at least one of: thermocouples, resistance temperature detectors, thermistors, infrared sensors. In an implementation, the at least one temperature sensor comprises a plurality of temperature sensors which enables to cover a broader area of the ocular surface of the user's eye from which the at least one point is selected.

Throughout the present disclosure, the term *"sensing area"* refers to a particular area within the ocular surface of the user's eye in which the at least one temperature sensor is able to measure the temperature of the at least one point on the ocular surface of the user's eye. Optionally, the sensing area of the at least one temperature sensor is in a shape of a circle, an oval, or any polygonal shape. Notably, the sensing area defines the boundaries on the ocular surface of the user's eye within which the at least one temperature sensor is able to operate. It will be appreciated that the sensing area for each temperature sensor amongst the at least one temperature sensor is different. Optionally, the largest dimension of the sensing area lies in a range of 5 millimetres to 30 millimetres. In this regard, the term *"largest dimension"* refers to a largest possible linear distance between two specific points on a periphery of the sensing area from amongst any possible linear distance between any corresponding two points on the periphery of the sensing area. For example, if the sensing area is in the shape of the circle, then the largest dimension of the sensing area is a diameter of the sensing area. Notably, if the largest dimension of the sensing area of the sensing area is less than 5 millimetres, then the sensing area becomes so small that the at least one point for measuring the temperature cannot be effectively selected within the sensing area. Moreover, if the largest dimension of the sensing area is more than 30 millimetres, then the sensing area becomes so large that is surpasses the ocular surface of the user's eyes which leads to interference from crosstalk values measured from points that don't lie on the ocular surface of the user's eye. More optionally, the largest dimension of the sensing area lies in a range of 9 millimetres to 25 millimetres. Yet more optionally, the largest dimension of the sensing area lies in a range of 10 millimetres to 15 millimetres. It will be appreciated that the largest dimension of the sensing area is dependent on a distance of the at least one temperature sensor from the user's eye. Optionally, the largest dimension of the sensing area is also dependent on a number of the at least one temperature sensor, wherein the largest dimension of the sensing area for each of the at least one temperature sensor decreases as the number of the at least one temperature sensors increases. Optionally, the largest dimension of the sensing area is also dependent on an age of the user, wherein the largest dimension of the sensing area is smaller when the user is child in comparison to the largest dimension of the sensing area when the user is an adult. For example, the largest dimension of the sensing area is 15 millimetres when the distance of the at least one temperature sensor is 14 millimetres from the user's eye. In another example, the largest dimension of the sensing area is 20 millimetres when the distance of the at least one temperature sensor is 18.7 millimetres from the user's eye. A technical effect of the largest dimension of the sensing area lying in the range of 5 millimetres to 30 millimetres is that the sensing area is precisely of the size that provides enough area to effectively select the at least one point for measuring the temperature, while significantly reducing unwanted crosstalk values.

Notably, the at least one point lying within the sensing area of the at least one temperature sensor implies that the at least one point on the ocular surface of the user's eye is selected such that the sensor arrangement is able to measure the temperature at each point amongst the at least one point on the ocular surface of the user's eye. Optionally, an orientation of the at least one temperature sensor is stationary at a fixed position which leads to the sensing area of the at least one temperature sensor being fixed in the ocular surface of the user's eye. Alternatively, the orientation of the at least one temperature sensor is movable which leads to the sensing area of the at least one temperature sensor to change its position in the ocular surface of the user's eye.

Optionally, the method further comprising:
processing eye-tracking data for tracking a gaze direction of the user's eye; and
detecting when the gaze direction of the user's eye coincides with a preset temperature measurement direction of the at least one temperature sensor,
wherein the step of measuring the temperature of the at least one point is performed at one or more time instants of said detection.

In this regard, the term *"gaze direction of the user's eye"* refers to a specific direction in which the user's eye is gazing at a given moment of time. Optionally, tracking the gaze direction of the user's eye comprises determining a position of at least one of: a pupil, an iris, of the user's eye, based on at least one of: the at least one eye image, the eye-tracking data collected by the eye-tracking unit. Optionally, the eye-tracking data is collected using the eye-tracking unit implemented by way of at least one of: contact lenses having sensors, cameras monitoring features of the user's eye. Such eye-tracking unit are well-known in the art. It will be appreciated that the eye-tracking data is indicative of the gaze direction of the user's eye and thus, processing the eye-tracking data enables to track the gaze direction of the user's eye. Optionally, the eye-tracking data is processed by employing at least one of: an image processing algorithm, a feature extraction algorithm, a data processing algorithm. Other suitable algorithm(s) can also be employed, depending on a type of the eye-tracking data.

Notably, tracking the gaze direction of the user's eye enables to keep a record of what is the gaze direction of the user's eye in real-time. Subsequently, tracking the gaze direction of the user's eye enables to detect when the gaze direction of the user's eye coincides with the preset temperature measurement direction of the at least one temperature sensor. Throughout the present disclosure, the term *"preset temperature measurement direction"* refers to a pre-determined direction from the at least one temperature sensor to the sensing area of the at least one temperature sensor on the user's eye. Optionally, the preset temperature measurement direction is fixed. It will be appreciated that when the gaze direction of the user's eye coincides with the preset temperature measurement direction, then the user's eye is facing the preset temperature measurement direction of the at least one temperature sensor, and thus, the pupil of the user's eye coincides with the sensing area of the at least one temperature sensor. Notably, performing the step of measuring the temperature of the at least one point at the one or more time instants of said detection implies that the temperature of the at least one point is measured at the one or more time instants when the sensing area of the at least one temperature sensor coincides with the pupil of the user's eye. A technical effect is that the temperature of the at least one point is measured at the one or more time instants when the sensing area of the at least one temperature sensor coincides with the pupil of the user's eye which improves an accuracy of the measured temperature.

Notably, the measured temperature of the at least one point has a mathematical relationship with core body temperature represented by a mathematical function. Subsequently, at each time instant amongst the plurality of the time instants, the mathematical function is used to estimate the core body temperature of the user at said time instant, based on the measured temperature of the at least one point at said time instant. For example, the core body temperature is estimated to be 36.5°C at a given time instant, based on measured temperature of three points that are 32.4°C, 32.7°C and 32.5°C at the given time instant. In an implementation, at the given instant of time amongst the plurality of time instants, the measured temperature of each point amongst the at least one point is used to determine an average measured temperature and subsequently, the core body temperature at the given time instant is estimated based on the average measured temperature, using the mathematical function. Similarly, the core body temperature is estimated at each time instant amongst the plurality of time instants. In another implementation, at the given instant of time amongst the plurality of time instants, the measured temperature of each point amongst the at least one point is used to estimate an individual corresponding core body temperature, based on the mathematical function. Subsequently, an average of each individual corresponding core body temperature is estimated as the core body temperature at the given time instant. Similarly, the core body temperature is estimated at each time instant amongst the plurality of time instants.

Optionally, the at least one point lies on at least one of: a pupil, an iris, a sclera, of the user's eye, wherein the core body temperature of the user is estimated using a first function, the first function indicating that the core body temperature is monotonically dependent on the measured temperature of the at least one point. In this regard, the term *"pupil"* refers to a central opening in the iris of the user's eye. Throughout the present disclosure, the term *"iris"* refers to a coloured portion of the user's eye that controls an amount of light entering the user's eye. Throughout the present disclosure, the term *"sclera"* refers to a white outer layer of the eyeball that surrounds the iris of the user's eye. It will be appreciated that when the at least one point lies on the at least one of: the pupil, the iris, the sclera, of the user's eye, then the mathematical relationship between the core body temperature and the measured temperature of the at least one point is such that the core body temperature is monotonically dependent on the measured temperature of the at least one point. Throughout the present disclosure, the term *"monotonically dependent"* refers to a type of mathematical relationship in which any increase or decrease in the measured temperature of the at least one point causes a corresponding increase or decrease in the core body temperature. Throughout the present disclosure, the term *"first function"* refers to that mathematical function which mathematically represents the core body temperature being monotonically dependent on the measured temperature of the at least one point. Optionally, the first function is represented as CBT = A + B*OST, wherein CBT is the core body temperature, OST is the measured temperature of the at least one point, and A and B are predefined constants determined from a large population size. A technical effect of estimating the core body temperature using the first function is that the core body temperature is accurately measured by effectively taking into account the core body temperature being monotonically dependent on the measured temperature of the at least one point.

Optionally, the method further comprising:
measuring an ambient temperature in a surrounding of the user's eye, at the plurality of time instants, using at least one ambient temperature sensor; and
adjusting the estimated core body temperature of the user, based on the ambient temperature.

In this regard, the term *"surrounding"* refers to an external real-world environment is which the user's eye is present. Throughout the present disclosure, the term *"ambient temperature"* refers to a temperature value of the surrounding of the user's eye. Notably, measuring the ambient temperature in the surrounding of the user's eye at the plurality of time instants ensures that the ambient temperature is taken into account for adjusting the estimated core body temperature of the user. Throughout the present disclosure, the term *"ambient temperature sensor"* refers to a temperature sensor that is specifically used for measuring the ambient temperature in the surrounding of the user's eye. Notably, adjusting the estimated core body temperature of the user, using the at least one ambient temperature sensor enables to negate any impact that the ambient temperature in the surrounding of the user's eye has on the estimated core body temperature of the user. Optionally, if the core body temperature is estimated based on the first function which is CBT = A + B*OST, wherein the CBT is the core body temperature, the OST is the measured temperature of the at least one point, and A and B are the predefined constants, then the estimated core body temperature is adjusted based on an equation which is CBT = A + B*OST - D(T_{amb} - T₀), wherein D is an experimentally derived constant, Tₐₘₚ is the ambient temperature, and T₀ is a reference temperature (for example, 25° C). A technical effect of the adjusting the measured temperature of the at least one point is that the impact of the ambient temperature in the surrounding of the user's eye on the measured temperature of the at least one point is successfully negated. Subsequently, the accuracy of the measured temperature of the at least one point is improved.

Optionally, the at least one point comprises a first set of points that lie on at least one of: a pupil, an iris, a sclera, of the user's eye, and a second set of points that lie on at least one of: an eyelid, a surrounding tissue, of the user's eye, and
wherein a first contribution of the first set of points to a measurement signal of the at least one temperature sensor lies within a predefined margin of a second contribution of the second set of points to the measurement signal, a given contribution of a given set of points being determined using a temperature-sensitivity distribution corresponding the sensing area and a number of points in the given set,
and wherein the method further comprises:
   processing eye-tracking data for determining a blink pattern of the user's eye;
   determining a first time instant and a second time instant of measuring the temperature of the at least one point, based on the blink pattern, wherein the first time instant lies within a blink interval and the second time instant lies within an inter-blink interval;
   determining a resultant measured temperature of the at least one point, based on the measured temperature of the at least one point at the first time instant and the second time instant,
wherein the core body temperature of the user is estimated using a second function, the second function indicating that the core body temperature is monotonically dependent on the resultant measured temperature of the at least one point.

In this regard, the term *"first set of points"* refers to a set of points amongst the at least one point that lie only within the corneal surface of the user's eye (i.e., on at least one of: the pupil, the iris, the sclera of the user's eye). Throughout the present disclosure, the term *"second set of points"* refers to another set of points amongst the at least one point that lie outside the corneal surface of the user's eye (i.e., on at least of: the eyelid, the surrounding tissue of the user's eye). Subsequently, a certain number of points (i.e., the first set of points) amongst the at least one point lie within the corneal surface of the user's eye, while remaining points (i.e., the second set of points) amongst the at least one point lie outside the corneal surface of the user's eye. Throughout the present disclosure, the term *"measurement signal"* refers to a signal that collectively comprises the measured temperature of each point amongst the at least one point. Herein, the given set of points is used to refer to either the first set of points or the second set of points at a given moment of time. Notably, the given contribution indicates how significant is the role of the given set of points in the measurement signal.

Throughout the present disclosure, the term *"temperature-sensitivity distribution"* refers to the measured temperature of each point, from amongst the at least one point, having a certain weightage based on a distance of said point from the preset temperature measurement direction. In other words, the temperature-sensitivity distribution refers to a weighted or quantitative mapping, across the sensing area of the at least one temperature sensor, of temperature measurements based on the distance of each measurement point from a preset temperature measurement direction. It may be appreciated that temperature-sensitivity distribution specifies how individual points (or groups of points) contribute to the overall measurement signal in response to local temperature variations. Notably, such mapping is not arbitrarily defined but is derived from systematic calibration procedures that account for both the spatial location and the intrinsic sensitivity of each measurement point.

Notably, the closer said point is to the preset temperature measurement direction, higher is the weightage of the measured temperature at said point, and vice versa. The given contribution of the given set of points being determined using the temperature-sensitivity distribution corresponding the sensing area and the number of points in the given set implies that how close or far the given set of points are from the preset temperature measurement direction, and how many points are in the at least one point determines how significant is the contribution of the given set of points to the measurement signal. Beneficially, the temperature-sensitivity distribution allows determining the contribution of each measurement point to the resultant temperature, with points closer to the preset direction having a higher weightage. In this regard, it is worth appreciating that by assigning a temperature-sensitivity value to each point (or set of points), the system can accurately evaluate the significance of temperature variations across a nonuniform sensing area, by reducing systematic measurement errors. Thereby, making the disclosed system inventive over conventional temperature sensors that function based on uniformly distributed sensitivity. Moreover, the temperature-sensitivity distribution allows for dynamic adjustment of sensor calibration and error compensation in real-time.

Optionally, the temperature-sensitivity distribution is used to determine contribution of two or more sets of measurement points from a preset temperature measurement direction, and selecting the set with a higher contribution for core body temperature estimation. Beneficially, in such case, the temperature-sensitivity distribution provides improved accuracy and consistency in temperature measurement.

Throughout the present disclosure, the term *"predefined margin"* refers to a numerical value that sets an upper limit for a maximum possible difference between the first contribution (i.e., the contribution of the first set of points to the measurement signal) and the second distribution (i.e., the contribution of the second set of points to the measurement signal). Notably, the first contribution lying within the predefined margin of the second contribution implies that the first contribution is close enough to the second contribution to ensure that first distribution is not negligible in comparison to the second contribution, and vice versa. Optionally, the predefined margin lies in a range of 0 to 10 percent.

Throughout the present disclosure, the term *"blink interval"* refers to a time duration of the blink of the user's eye. It may be appreciated that during the blink interval the eyes of the user is partially or fully closed, i.e., the user is in a state where the eyelid is partially or fully closed, preventing direct visibility of the ocular surface. Notably, the blink pattern is indicative of the blink interval and the inter-blink interval which enables to determine the first time instant and the second time instant such that the first time instant lies within the blink interval, i.e., when the eyes are closed; and the second time instant lies within the inter-blink interval, i.e., when the eyes are open. The first time instant lying within the blink interval enables to measure the temperature of the second set of points at the first time instant, as the user's eye is partially or fully closed during the blink interval. The second time instant lying within the inter-blink interval enables to measure the temperature of the first set of points at the second time instant, as the user's eye is open. It may be appreciated that the eye region exhibits distinct thermal behaviors when open and closed (partially or fully). Notably, when the eye is open, the measured temperature is influenced by heat dissipation due to exposure to ambient air, thus, resulting in a lower temperature reading compared to the actual core body temperature. Similarly, when the eye is partially or fully closed, the measured temperature is influenced by heat retention, leading to a higher temperature reading that is closer to the core body temperature but still affected by surface-level thermal effects. By explicitly measuring the temperature in both states, namely the resultant measured temperature, the system captures a more comprehensive thermal profile of the user at a given point from amongst the at least one point.

Throughout the present disclosure, the term *"resultant measured temperature of the at least one point"* refers to a final temperature value of the at least one point that is determined after taking into account the first contribution of the first set of points on the measured temperature of the at least one point at the second time instant, and the second contribution of the second set of points on the measured temperature of the at least one point at the first time instant. Beneficially, by combining said measurements using the second function, the system compensates for the thermal dynamics of the eye region, by not being biased by transient effects of single states of eye.

Optionally, the resultant measured temperature of the at least one point is determined by one of: calculating an average, calculating a weighted average, performing an interpolation, of the measured temperature of the at least one point at the first time instant and the second time instant, and the like. Subsequently, calculation of the core body temperature becomes monotonically dependent on the resultant measured temperature of the at least one point. Throughout the present disclosure, the term *"second function"* refers to that mathematical function which mathematically represents the core body temperature being monotonically dependent on the resultant measured temperature of the at least one point. For example, the second function may assign different weights to the open-eye and closed-eye measurements based on their respective contributions to the core body temperature signal. A technical effect is that the measurement of the temperature of the at least one point is effectively adjusted for different positions of the at least one point and different time instants for measuring the temperature of the at least one point, which significantly enhances an applicability of the aforementioned system. Moreover, the combined measurements allows dynamically adjusting the temperature estimation process based on the physiological state of the measurement site, namely, when the eyes are open and when the eyes are partially or fully closed. Therefore, the combined measurements enables the system to overcome limitations of conventional single-point or single-state temperature measurement systems. The combined measurements reduce the impact of random errors or noise (such as changes in ambient temperature or user movement) in the temperature readings, improving the reliability of the estimation.

Optionally, the second function may be derived from empirical data, thermal modeling, or machine learning algorithms, ensuring that the resultant measured temperature accurately represents the user's core body temperature.

Optionally, the second function may be adapted for individual variations in thermal response, such as differences in skin thickness, blood flow, or ambient conditions. Beneficially, such adaptability ensures that the system provides accurate results across a wide range of users and environments.

Optionally, the at least one point comprises a first set of points that lie on at least one of: a pupil, an iris, a sclera, of the user's eye, and a second set of points that lie on at least one of: an eyelid, a surrounding tissue, of the user's eye, and
wherein a second contribution of the second set of points to a measurement signal of the at least one temperature sensor is greater than a first contribution of the first set of points to the measurement signal by at least a threshold value, a given contribution of a given set of points being determined using a temperature-sensitivity distribution corresponding the sensing area and a number of points in the given set, and wherein the step of measuring the temperature of the at least one point comprises measuring a temperature of the points in the second set, and wherein the core body temperature of the user is estimated using a third function, the third function indicating that the core body temperature is linearly dependent on the measured temperature of the points in the second set.

In this regard, the term *"threshold value"* refers to a numerical value that indicates a significantly high difference between the second contribution and the first contribution. Notably, the second contribution being greater than the first contribution by at least the threshold value indicates that the difference between the second contribution and the first contribution is so significantly high that the first contribution is negligible is comparison to the second contribution. Notably, the step of measuring the temperature of the at least one point comprises only measuring the temperature of the points in the second set of points and does not comprise measuring the temperature of the first set of points as the first contribution of the first set of points is negligible in comparison to the second contribution of the second set of points. Subsequently, the core body temperature becomes linearly dependent on the measured temperature of the points in the second set, based on the mathematical relationship between the core body temperature and the measured temperature of the at least one point. In an example, the measured temperature of the points in the second set of points are 33.3°C for a first point amongst the second set points and 31.5°C for a second point amongst the second set. It will be appreciated that the mathematical relationship of being linearly dependent is well known in the art. Throughout the present disclosure, the term *"third function"* refers to that mathematical function which mathematically represents the core body temperature being linearly dependent on the measured temperature of the points in the second set. For example, the third function is represented as CBT = C * DOST, wherein CBT is the core body temperature, DOST is the measured temperature of the points in the second set, and C is a predefined constant value determined from a large population size. A technical effect is that the core body temperature is accurately estimated by effectively adjusting the measured temperature of the at least one point when the second contribution of the second set of points is significantly greater than the first contribution of the first set of points. By comparing measurement point contributions to a threshold value, using a third function, the system excludes point with low relevance or high noise, ensuring more reliable and accurate data is used for measuring the core body temperature.

Optionally, the method further comprising determining a core body temperature trend in the time period, based on the estimated core body temperature of the user across the plurality of time instants. Throughout the present disclosure, the term *"core body temperature trend"* refers to a pattern that indicates how the core body temperature of the user changes during the time period. The core body temperature trend in the time period being determined based on the estimated core body temperature of the user across the plurality of time instants enables to accurately reflect how the core body temperature of the user changes at the plurality of time instants within the time period. Optionally, the core body temperature trend is represented in form of a graphical representation. A technical effect of determining the core body temperature trend is that significant insights into a functioning of the user's body during the time period can be determined based on the core body temperature trend.

Optionally, when the estimated core body temperature is greater than a predefined threshold value, the method further comprises generating at least one indication indicating that the estimated core body temperature is greater than the predefined threshold value. In this regard, the term *"predefined threshold value"* refers to a maximum value for the core body temperature that can be accurately estimated using the mathematical relationship between the core body temperature and the measured temperature of the at least one point, as the measured temperature of the at least one point ceases to increase beyond a certain value (preferably between 36.5°C to 37°C). Notably, the estimated core body temperature being greater than the predefined threshold value indicates the estimated core body temperature to be possibly unreliable. Throughout the present disclosure, the term *"at least one indication"* refers to one or more warning signals that informs the user about the possible unreliability in the estimated core body temperature due to the estimated core body temperature being greater than the predefined threshold value. Optionally, the at least one indication is generated in form a text indication (e.g., a message), a sound indication (e.g., a beeping signal), a visual indication (e.g., blinking of lights) and the like. A technical effect of generating the at least one indication is that the user is effectively made aware of the possible unreliability in the estimated core body temperature when the estimated core body temperature is greater than the predefined threshold value.

Therefore, the technical problem addressed by the system of the present disclosure is how to improve the accuracy and reliability of the core body temperature estimation using non-invasive methods. In this regard, transient thermal effects and noise are compensated in the measurement signal; most relevant measurement points are dynamically selected for temperature estimation; and a robust estimation method is provided that adapts to user-specific and environmental variations. Specifically, contributions of different sets of points to a measurement signal are compared; and the second set of points is selected if its contribution exceeds the first set by a threshold value, to ensure that only the most relevant and reliable data is used for core body temperature estimation. In this regard, a third function is specifically tailored, to account for spatial and temporal variations in the sensing area, to improve the accuracy of the estimation of the core body temperature. Moreover, the system dynamically adapts to variations in the sensing area and user-specific factors, ensuring consistent and accurate measurements.

The present disclosure also relates to the optical apparatus as described above. Various embodiments and variants disclosed above, with respect to the aforementioned method, apply *mutatis mutandis* to the optical apparatus.

Notably, the frame is employed to hold at least one optical element (for example, an optical lens) per eye of the user. It will be appreciated that a material of the frame could be plastic, metal, polymer, and the like. Moreover, the frame may be lightweight, ergonomically designed, and easy to use. It will be appreciated that the optical apparatus is in use when the optical apparatus is worn on the user's head. Optionally, the at least one temperature sensor is arranged on one of: a rim, a bridge, of the frame, and the like.

Throughout the present disclosure, the term *"processor"* refers to a computational element that is operable to execute instructions of the optical apparatus. Examples of the at least one processor include, but are not limited to, a microprocessor, a microcontroller, a complex instruction set computing (CISC) microprocessor, a reduced instruction set (RISC) microprocessor, a very long instruction word (VLIW) microprocessor, or any other type of processing circuit. Furthermore, the at least one processor may refer to one or more individual servers, processing devices and various elements associated with a processing device that may be shared by other processing devices. Additionally, one or more individual processors, processing devices and elements are arranged in various architectures for responding to and processing the instructions that execute the instructions of the optical apparatus. Optionally, the at least one processor is coupled to the at least one temperature sensor in a wired or wireless manner. Notably, prior to estimating the core body temperature, the at least one processor is configured to receive sensor data from the at least one temperature sensor, the sensor data being indicative of the measured temperature of the at least one point.

Optionally, when the optical apparatus is in use, the at least one temperature sensor is arranged at a predefined distance from the user's eye, the predefined distance lying in a range of 5 millimetres to 30 millimetres from the user's eye.

Optionally, the at least one temperature sensor is capable of measuring infrared emissions from human skin which lie in a range of 1 micrometre to 20 micrometres. In this regard, the at least one temperature sensor being capable of measuring the infrared emissions from the human skin enables the at least one temperature sensor to measure the temperature of any point amongst the at least one point that lies on at least one of: an eyelid, a surrounding tissue, of the user's eye in a non-contact manner. A technical effect of the at least one temperature sensor being capable of measuring the infrared emissions from the human skin in the range of 1 micrometres to 20 micrometres is that the temperature of any point amongst the at least one point that lies on at least one of: the eyelid, the surrounding tissue, of the user's eye is also effectively and accurately measured.

Optionally, the at least one temperature sensor is at least one of: a pyroelectric temperature sensor, an Indium Gallium Arsenide (InGaAs)-based temperature sensor, a thermopile temperature sensor. In this regard, the pyroelectric temperature sensor, the Indium Gallium Arsenide (InGaAs)-based temperature sensor, and the thermopile temperature sensor are well-known in the art. Notably, the pyroelectric temperature sensor is able to measure the infrared emissions between a range of 12 to 14 micrometres, and the InGaAs-based temperature sensor is able to measure the infrared emissions up to 2.5 micrometres. Optionally, when the at least one temperature sensor is at least one pyroelectric temperature sensor, then the at least one temperature sensor further comprises at least one optical shutter arranged on an optical path between the eye and a light-sensitive surface of the at least one pyroelectric temperature sensor. A technical effect is that the at least one temperature sensor is accurate, cost effective and reliable to use.

Optionally, the optical apparatus further comprises at least one of:
an eye-tracking unit that in operation, collects eye-tracking data;
at least one ambient temperature sensor that in operation, measures an ambient temperature in a surrounding of the user's eye, wherein the at least one processor is coupled to the at least one of: the eye-tracking unit, the at least one ambient temperature sensor.

### DETAILED DESCRIPTION OF THE DRAWINGS

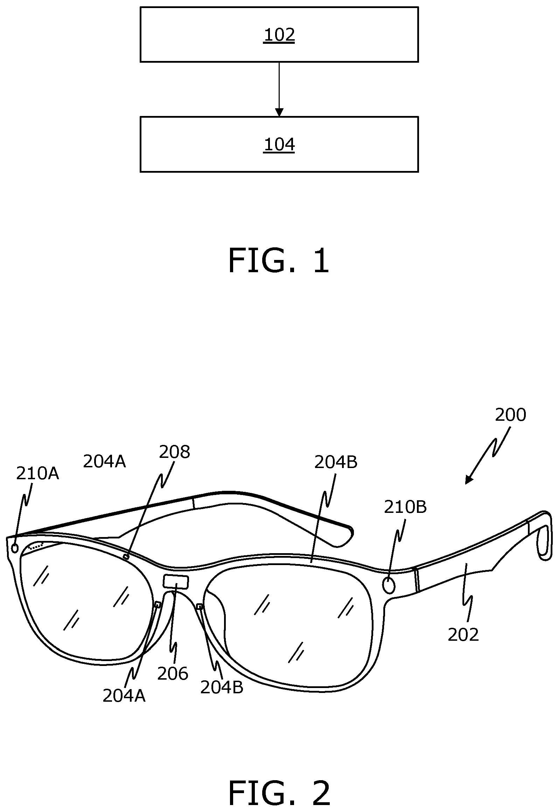

Referring to FIG. 1, illustrated is a flowchart for depicting steps of a method for tracking a core body temperature of a user using an optical apparatus, in accordance with an embodiment of the present disclosure. At step **102,** a temperature of at least one point on an ocular surface of the user's eye is measured, at a plurality of time instants during a time period, wherein said measuring is performed using at least one temperature sensor, and wherein the at least one point lies within a sensing area of the at least one temperature sensor. At step **104,** the core body temperature of the user is estimated at the plurality of time instants, based on the measured temperature of the at least one point at the plurality of time instants.

The aforementioned steps are only illustrative and other alternatives can also be provided where one or more steps are added, one or more steps are removed, or one or more steps are provided in a different sequence without departing from the scope of the claims herein.

Referring to FIG. 2, illustrated is a schematic illustration of an optical apparatus **200,** in accordance with an embodiment of the present disclosure. As shown, the optical apparatus **200** comprises a frame **202.** Moreover, the optical apparatus **200** comprises at least one temperature sensor (depicted as a first temperature sensor **204A,** and a second temperature sensor **204B)** arranged on the frame **202,** wherein when the optical apparatus **200** is in use, the at least one temperature sensor **204A** and **204B** faces a user's eye. Furthermore, the optical apparatus **200** comprises at least one processor (depicted as a processor **206)** coupled to the at least one temperature sensor **204A** and **204B,** wherein the at least one processor **206** is configured to control the at least one temperature sensor **204A** and **204B** to measure a temperature of at least one point on an ocular surface of a user's eye, at a plurality of time instants during a time period, wherein the at least one point lies within a sensing area of the at least one temperature sensor **204A** and **204B.** Moreover, the at least one processor **206** is configured to estimate the core body temperature of the user at the plurality of time instants, based on the measured temperature of the at least one point at the plurality of time instants. Optionally, the optical apparatus **200** further comprises at least one of: an eye-tracking unit **208** that in operation, collects eye-tracking data; at least one ambient temperature sensor (depicted as a first ambient temperature sensor **210A** and a second ambient temperature sensor **210B)** that in operation, measures an ambient temperature in a surrounding of the user's eye, wherein the at least one processor **206** is coupled to the at least one of: the eye-tracking unit **208** the at least one ambient temperature sensor **210A** and **210B.**

FIG. 2 is merely an example, which should not unduly limit the scope of the claims herein. It is to be understood that the specific implementations of the optical apparatus **200** are provided as examples and are not to be construed as limiting it to specific numbers, sizes, or shapes of the at least one temperature sensor **204A-B,** the at least one processor **206,** the eye-tracking unit **208,** the at least one ambient temperature sensor **210A-B,** and similar. A person skilled in the art will recognize many variations, alternatives, and modifications of embodiments of the present disclosure.

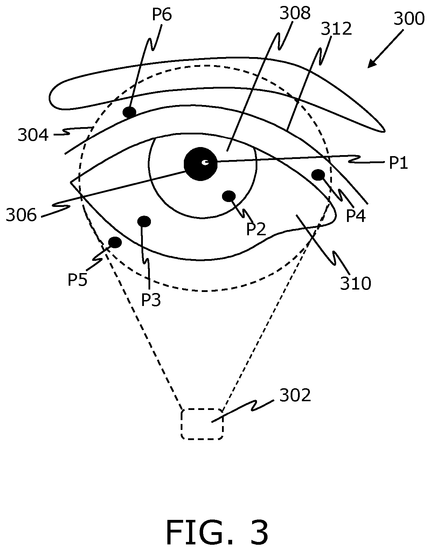

Referring to FIG. 3, illustrated is a schematic illustration of a user's eye **300** when an optical apparatus in in use, in accordance with an embodiment of the present disclosure. As shown, a temperature of at least one point (depicted as a first point **P1,** a second point **P2,** a third point **P3,** a fourth point **P4,** a fifth point **P5,** and a sixth point **P6)** on an ocular surface of a user's eye **300** is measured, at a plurality of time instants during a time period, wherein the at least one point lies within a sensing area **304** of at least one temperature sensor (depicted as a first temperature sensor **302).** Optionally, the at least one point **P1, P2, P3, P4, P5,** and **P6** comprises a first set of points (depicted as the first point **P1,** the second point **P2,** and the third point **P3)** that lie on at least one of: a pupil **306,** an iris **308,** a sclera **310,** of the user's eye **300,** and a second set of points (the fourth point **P4,** the fifth point **P5,** and the sixth point **P6)** that lie on at least one of: an eyelid **312,** a surrounding tissue, of the user's eye **300.**

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A method for tracking a core body temperature of a user using an optical apparatus (200), the method comprising:
measuring a temperature of at least one point (P1, P2, P3, P4, P5, P6) on an ocular surface of the user's eye, at a plurality of time instants during a time period, wherein said measuring is performed using at least one temperature sensor (204A, 204B, 302), and wherein the at least one point lies within a sensing area (304) of the at least one temperature sensor; and
estimating the core body temperature of the user at the plurality of time instants, based on the measured temperature of the at least one point at the plurality of time instants.

2. A method according to claim 1, further comprising:
processing eye-tracking data for determining a blink pattern of the user's eye; and
determining a time instant of measuring the temperature of the at least one point (P1, P2, P3, P4, P5, P6), based on the blink pattern, wherein said time instant lies within an inter-blink interval.

3. A method according to claim 1 or 2, further comprising:
processing eye-tracking data for tracking a gaze direction of the user's eye; and
detecting when the gaze direction of the user's eye coincides with a preset temperature measurement direction of the at least one temperature sensor (204A, 204B, 302),
wherein the step of measuring the temperature of the at least one point (P1, P2, P3, P4, P5, P6) is performed at one or more time instants of said detection.

4. A method according to any of the preceding claims, further comprising:
measuring an ambient temperature in a surrounding of the user's eye, at the plurality of time instants, using at least one ambient temperature sensor; and
adjusting the estimated core body temperature of the user, based on the ambient temperature.

5. A method according to any of the preceding claims, further comprising determining a core body temperature trend in the time period, based on the estimated core body temperature of the user across the plurality of time instants.

6. A method according to any of the preceding claims, wherein the largest dimension of the sensing area (304) lies in a range of 5 millimetres to 30 millimetres.

7. A method according to any of the preceding claims, wherein the at least one point (P1, P2, P3, P4, P5, P6) lies on at least one of: a pupil (306), an iris (308), a sclera (312), of the user's eye, and wherein the core body temperature of the user is estimated using a first function, the first function indicating that the core body temperature is monotonically dependent on the measured temperature of the at least one point.

8. A method according to any of claims 1-6, wherein the at least one point (P1, P2, P3, P4, P5, P6) comprises a first set of points (P1, P2, P3) that lie on at least one of: a pupil (306), an iris (308), a sclera (310), of the user's eye, and a second set of points (P4, P5, P6) that lie on at least one of: an eyelid (312), a surrounding tissue, of the user's eye, and
wherein a first contribution of the first set of points to a measurement signal of the at least one temperature sensor (204A, 204B, 302) lies within a predefined margin of a second contribution of the second set of points to the measurement signal, a given contribution of a given set of points being determined using a temperature-sensitivity distribution corresponding the sensing area (304) and a number of points in the given set,
and wherein the method further comprises:
processing eye-tracking data for determining a blink pattern of the user's eye;
determining a first time instant and a second time instant of measuring the temperature of the at least one point, based on the blink pattern, wherein the first time instant lies within a blink interval and the second time instant lies within an inter-blink interval;
determining a resultant measured temperature of the at least one point, based on the measured temperature of the at least one point at the first time instant and the second time instant,
wherein the core body temperature of the user is estimated using a second function, the second function indicating that the core body temperature is monotonically dependent on the resultant measured temperature of the at least one point.

9. A method according to any of claims 1-6, wherein the at least one point (P1, P2, P3, P4, P5, P6) comprises a first set of points (P1, P2, P3) that lie on at least one of: a pupil (306), an iris (308), a sclera (310), of the user's eye, and a second set of points (P4, P5, P6) that lie on at least one of: an eyelid, a surrounding tissue, of the user's eye, and wherein a second contribution of the second set of points to a measurement signal of the at least one temperature sensor (204A, 204B, 302) is greater than a first contribution of the first set of points to the measurement signal by at least a threshold value, a given contribution of a given set of points being determined using a temperature-sensitivity distribution corresponding the sensing area (304) and a number of points in the given set,
and wherein the step of measuring the temperature of the at least one point comprises measuring a temperature of the points in the second set, and wherein the core body temperature of the user is estimated using a third function, the third function indicating that the core body temperature is linearly dependent on the measured temperature of the points in the second set.

10. A method according to any of the preceding claims, wherein when the estimated core body temperature is greater than a predefined threshold value, the method further comprises generating at least one indication indicating that the estimated core body temperature is greater than the predefined threshold value.

11. An optical apparatus (200) comprising:
a frame (202);
at least one temperature sensor (204A, 204B, 302) arranged on the frame, wherein when the optical apparatus is in use, the at least one temperature sensor faces a user's eye; and
at least one processor (206) coupled to the at least one temperature sensor, wherein the at least one processor is configured to:
control the at least one temperature sensor to measure a temperature of at least one point (P1, P2, P3, P4, P5, P6) on an ocular surface of a user's eye, at a plurality of time instants during a time period, wherein the at least one point lies within a sensing area (304) of the at least one temperature sensor; and
estimate the core body temperature of the user at the plurality of time instants, based on the measured temperature of the at least one point at the plurality of time instants.

12. An optical apparatus (200) of claim 11, wherein when the optical apparatus is in use, the at least one temperature sensor (204A, 204B, 302) is arranged at a predefined distance from the user's eye, the predefined distance lying in a range of 5 millimetres to 30 millimetres from the user's eye.

13. An optical apparatus (200) of any of claims 11 or 12, wherein the at least one temperature sensor (204A, 204B, 302) is capable of measuring infrared emissions from human skin which lie in a range of 1 micrometre to 20 micrometres.

14. An optical apparatus (200) of any of claims 11-13, wherein the at least one temperature sensor (204A, 204B, 302) is at least one of: a pyroelectric temperature sensor, an Indium Gallium Arsenide (InGaAs)-based temperature sensor, a thermopile temperature sensor.

15. An optical apparatus (200) of any of claims 11-14, further comprising at least one of:
an eye-tracking unit (208) that in operation, collects eye-tracking data;
at least one ambient temperature sensor (210A, 210B) that in operation, measures an ambient temperature in a surrounding of the user's eye,
wherein the at least one processor (206) is coupled to the at least one of: the eye-tracking unit, the at least one ambient temperature sensor.
